(19) Europäisches Patentamt
European Patent Office
Office européen des brevets

(11) Veröffentlichungsnummer: **0 521 496 B1**

(12) **EUROPÄISCHE PATENTSCHRIFT**

(45) Veröffentlichungstag der Patentschrift: **19.10.94**

(51) Int. Cl.$^{5}$: **C07D 307/24**, C07D 309/08, C07D 333/38, C07D 335/02, C09K 19/54

(21) Anmeldenummer: **92111186.0**

(22) Anmeldetag: **02.07.92**

# (54) Verfahren zur Herstellung optisch aktiver Carbonsäuren.

(30) Priorität: **04.07.91 DE 4122218**

(43) Veröffentlichungstag der Anmeldung:
**07.01.93 Patentblatt 93/01**

(45) Bekanntmachung des Hinweises auf die Patenterteilung:
**19.10.94 Patentblatt 94/42**

(84) Benannte Vertragsstaaten:
**BE CH DE FR GB IT LI NL**

(56) Entgegenhaltungen:
**EP-A- 0 367 515**
**EP-A- 0 382 506**

**BIOCHEMISTRY. Bd. 5, Nr. 12, 1966, Easton, PA (US); J.R. RAPP et al., Seiten 4100-4105&NUM;**

**JOURNAL OF ORGANIC CHEMISTRY. Bd. 27, März 1962, Easton, PA (US); R.K. HILL et al., Seiten 921-924&NUM;**

(73) Patentinhaber: **Consortium für elektrochemische Industrie GmbH**
**Zielstattstrasse 20**
**D-81379 München (DE)**

(72) Erfinder: **Fritz-Langhals, Elke, Dr.**
**Kleiststrasse 16**
**W-8012 Ottobrunn (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

CHEMICAL ABSTRACTS, Band 91, Nr. 7, 13 August 1979, Columbus, OH (US);S; C. GRIECO et al., Nr. 51756n&NUM;

JOURNAL OF THE AMERICAN CHEMICAL SOCIETY, Bd. 99, Nr. 11, 25 Mai 1977, Gaston, PA (US); G. STORK et al., Seiten 3851-3853&NUM;

COLLECTION OF CZECHOSLOVAK CHEMICAL COMMUNICATIONS. Bd. 51, 1986, PRAG (CR); O. CERVINKA et al., Seiten 404-407&NUM;

CANADIAN JOURNAL OF CHEMISTRY, Bd. 61, 1983, OTTAWA (CA); P.C. BELANGER et al., Seiten 1383-1386&NUM;

CHEMICAL ABSTRACTS, Bd. 112, Nr. 17, 23 April 1990, Columbus, OH (US); H. NODAIRA et al., Nr. 158037h&NUM;

**Beschreibung**

Die Erfindung betrifft ein Verfahren zur Herstellung optisch aktiver Carbonsäuren der allgemeinen Formeln I* oder II*

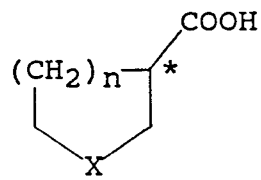

oder

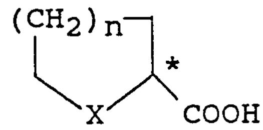

I*

II*

in denen X ein Sauerstoff- oder Schwefelatom und n 1 oder 2 sein kann.

Zur Darstellung optisch aktiver Carbonsäuren durch Racematspaltung wurde bisher üblicherweise die zu trennende racemische Carbonsäure mit einem optisch aktiven Amin umgesetzt und die gebildeten diastereomeren Salze durch fraktionierte Kristallisation getrennt. Diese Methode wurde auch bei der Darstellung einiger der obengenannten optisch aktiven heterocyclischen Carbonsäuren angewandt.

So beschreiben Bélanger et al. (Can. J. Chem. 61 (1983) 1383) ein Verfahren zur Darstellung von (R)- bzw. (S)-Tetrahydrofuran-2-carbonsäure (Formel II*: n = 1, X = O) mit Hilfe von Brucin bzw. Cinchonidin. Diese Reagenzien können aber aufgrund ihrer Giftigkeit und des hohen Preises für die Gewinnung größerer Substanzmengen nicht eingesetzt werden. Cervinka et al. (Collect. Czech. Chem. Commun. 51 (1986) 404) beschreiben die Racematspaltung von Tetrahydrofuran-2-carbonsäure mit Chinin. Mit dieser Methode wird jedoch nur das (S)-Enantiomere erhalten, dessen Ausbeute nach der erforderlichen mehrfachen Umkristallisation nur etwa 10% beträgt. In der JP-A 01/216983 wird die Racematspaltung von Tetrahydrofuran-2-carbonsäure mit Hilfe von optisch aktivem 4-Brom- oder 4-Chlor-1-phenylethylamin beschrieben. Die Herkunft dieser optisch aktiven Amine ist jedoch nicht bekannt.

Die Racematspaltung von Tetrahydrothiophen-2-carbonsäure (Formel II: n = 1, X = S) mit Hilfe von Brucin oder Cinchonidin (Claeson et al., Ark. Kemi 26 (1967) 247; Stork und Kreft III, J. Am. Chem. Soc. 99 (1977) 3851) und auch mit Hilfe von N-Methyl-(S)-1-phenyl-ethylamin (Cervinka et al., Collect. Czech. Chem. Commun. 51 (1986) 404) lieferte nur jeweils eines der beiden Enantiomeren in mäßigen Ausbeuten. Gleiches gilt auch für die Racematspaltung von (2H)-Tetrahydropyran-2-carbonsäure (Formel II: n = 2, X = O) mit Chinin ((S)-Enantiomeres in 4 % Ausb., Cervinka et al., Collect. Czech. Chem. Commun. 51 (1986) 404), die Racematspaltung von Tetrahydrofuran-3-carbonsäure (Formel I: n = 1, X = O) mit Chinin (Katsura, Chem. Abstr. 56 (1962) 9950; Kaneko et al., Chem & Ind. 1960, 1187; Hill et al. J. Org. Chem. 27, (1962) 921) sowie die Racematspaltung von Tetrahydrothiophen-3-carbonsäure (Formel I: n = 1, X = S) mit Hilfe von N-Methyl-(S)-1-phenylethylamin (Cervinka et al., Collect. Czech. Chem. Commun. 51 (1986) 404).

Vorstehende Nachteile stehen der Darstellung größerer Substanzmengen im Wege und schränken die Möglichkeit der Anwendung von optisch aktiven heterocyclischen Carbonsäuren als Synthesebausteine, etwa in Flüssigkristallen, ein (vgl. EP-A 355561). Überdies wurden (2H)-Tetrahydrothiopyran-2-carbonsäure (Formel II: n = 2, X = S), (2H)-Tetrahydropyran-3-carbonsäure (Formel I: n = 2, X = O) und (2H)-Tetrahydrothiopyran-3-carbonsäure (Formel I, n = 2, X = S) bislang noch nicht in optisch aktiver Form dargestellt. Es besteht aber ein Bedarf an diesen zuletzt genannten bislang unbekannten Carbonsäuren, da sie aufgrund ihrer Strukturverwandtschaft mit den oben erwähnten bekannten Carbonsäuren ebenfalls als Bausteine für Flüssigkristalle dienen können.

Es bestand daher die Aufgabe, ein einfaches Verfahren zur Gewinnung der optisch aktiven Verbindungen mit den Formeln I* und II* zur Verfügung zu stellen, mit welchem die optischen Antipoden auf einfache Weise in guten Ausbeuten gewonnen werden können.

Diese Aufgabe wird durch das erfindungsgemäße Verfahren gelöst, bei dem zur Racematspaltung der Carbonsäuren anstelle der üblichen Trennung durch Kristallisation diastereomerer Salze eine Trennung der kovalenten Verknüpfungsprodukte zwischen racemischer Carbonsäure und optisch aktivem Amin erfolgt. Anstelle der üblicherweise verwendeten optisch aktiven Amine werden hierfür optisch aktive 2-Aminocarbonsäureester eingesetzt. Überraschenderweise hat sich nämlich gezeigt, daß Carbonsäureamide, die durch Verknüpfung von optisch aktiven 2-Aminocarbonsäureestern mit racemischen Carbonsäuren der allgemeinen Formeln I oder II über eine Amidbindung hergestellt wurden, besonders für eine Diastereomerentrennung zur Darstellung der optischen Antipoden der Carbonsäuren geeignet sind. Die Diastereomerentrennung gelingt mit vorstehenden Carbonsäureamiden in besonders einfacher Weise und in hohen Ausbeuten.

Gegenstand der vorliegenden Erfindung ist ein Verfahren zur Herstellung optisch aktiver Carbonsäuren der allgemeinen Formeln I* oder II*,

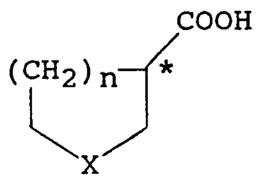

oder

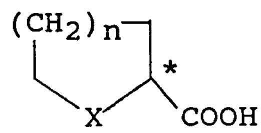

I* II*

in denen X ein Sauerstoff- oder Schwefelatom und n 1 oder 2 ist, dadurch gekennzeichnet, daß eine racemische Carbonsäure I oder II oder deren Derivate mit einem optisch aktiven 2-Aminocarbonsäureester zu den diastereomeren Carbonsäureamiden umgesetzt wird, die Diastereomeren getrennt werden und nach Spaltung der Amidbindung die optisch aktiven Carbonsäuren der allgemeinen Formeln I* oder II* isoliert werden.

Die racemischen Carbonsäuren der allgemeinen Formeln

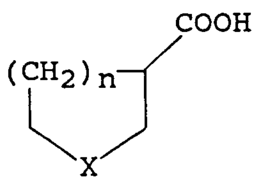

oder

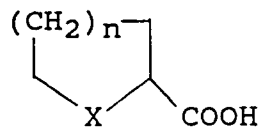

I II

mit X = O oder S und n = 1 oder 2 können nach literaturbekannten Methoden hergestellt werden.

Tetrahydrofuran-2-carbonsäure (Formel II: n = 1, X = O) kann bei spielsweise durch Hydrierung von Furan-2-carbonsäure mit Raney-Nickel hergestellt werden (Paul und Hilly, Compt. rend. 208 (1939) 359). Tetrahydrothiophen-2-carbonsäure (Formel II: n = 1, X = S) ist durch Umsetzung von 2,5-Dibromvaleriansäuremethylester mit Natriumsulfid und anschließender Verseifung der Estergruppe zugänglich (Claeson und Jonsson, Ark. Kemi 26 (1967) 247), (2H)-Tetrahydropyran-2-carbonsäure (Formel II: n = 2, X = O) kann durch Oxidation von (2H)-2-Hydroxymethyl-tetrahydropyran mit Chromtrioxid erhalten werden (Pasto und Serve, J. Am. Chem. Soc. 87 (1965) 1515), (2H)-Tetrahydrothiopyran-2-carbonsäure (Formel II: n = 2, X = S) ist beispielsweise aus 2,6- Dibromhexansäure durch Ringschluß mit Natriumsulfid herstellbar (Roush et al., J. Am. Chem. Soc. 101 (1979) 2971).

Tetrahydrofuran-3-carbonsäure (Formel I: n = 1, X = O) kann durch Hydrierung von Furan-3-carbonsäure mit Raney-Nickel dargestellt werden (Boekelheide und Morrison, J. Am. Chem. Soc. 80 (1958) 3905), Tetrahydrothiophen-3-carbonsäure (Formel I: n = 1, X = S) ist durch Umsetzung von 1,4-Dibrombutan-2-carbonsäuremethylester mit Natriumsulfid und anschließender Verseifung der Estergruppe zugänglich (Kapovits und Kucsman, Acta Chim. Acad. Sci. Hung. 34 (1962) 79), (2H)-Tetrahydropyran-3-carbonsäure (Formel I: n = 2, X = O) kann durch Hydrierung von 2,3-Dihydro-(4H)-pyran-5-carbonsäure mit Raney-Nickel erhalten werden (Silberman, J. Org. Chem. 25 (1960) 151), (2H)-Tetrahydrothiopyran-3-carbonsäure (Formel I: n = 2, X = S) ist beispielsweise aus 1,5-Dibrompentan-2-carbonsäure-ethylester durch Ringschluß mit Natriumsulfid und anschließender Verseifung herstellbar (Kapovits und Kucsman, Acta Chim. Acad. Sci. Hung. 34 (1962) 79).

Für die Umsetzung mit den optisch aktiven 2-Aminocarbonsäureestern können außer den racemischen Carbonsäuren der allgemeinen Formeln I oder II auch Derivate derselben wie Säurehalogenide, insbesondere Säurechloride, Ester, Anhydride, oder Amide eingesetzt werden. Vorzugsweise finden die Säurechloride der racemischen Carbonsäuren der allgemeinen Formeln I oder II Verwendung. Die Herstellung der

Säurechloride aus den racemischen Carbonsäuren erfolgt vorzugsweise unter Verwendung von Thionylchlorid (Wróbel und Hejchman, Synthesis 1987, 452).

Als optisch aktive Aminkomponente eignen sich (S)- oder (R)-2-Aminocarbonsäureester der allgemeinen Formel III*,

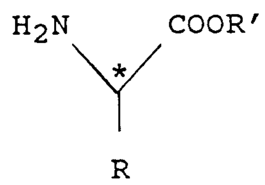

III*

wobei der Rest R ein linearer, verzweigter oder cyclischer $C_{1-6}$-Alkylrest ist, bei dem gegebenenfalls ein Kohlenstoffatom durch ein Sauerstoff- oder Schwefelatom oder durch den Rest >NR"ersetzt ist, wobei R" einen Methyl- oder Ethylrest bedeutet oder R ein linearer, verzweigter oder cyclischer $C_{1-6}$-Alkylrest ist, der mit den Resten -OH, -SH, $-SCH_3$, $-NH_2$, -COOR"', $-CONH_2$ oder durch eine Phenylgruppe, die gegebenenfalls mit $-CH_3$, -OH oder $-OCH_3$ substituiert ist, substituiert ist, wobei R"' eine Methyl-, Ethyl-, n-Propyl- oder i-Propylgruppe bedeutet, oder der Rest R eine Phenylgruppe bedeutet, die unsubstituiert oder mit $-CH_3$, -OH oder $-OCH_3$ substituiert ist. Der Rest R' ist ein linearer, verzweigter oder cyclischer $C_{1-20}$-Alkyl- oder $C_{1-20}$-Alkoxyalkylrest, der gegebenenfalls mit einer Phenylgruppe substituiert ist oder ein Phenylrest.

Bevorzugte Reste R sind lineare oder verzweigte $C_{1-6}$-Alkyl reste, $-CH_2OH$, $-CHOH(CH_3)$, $-CH_2SH$, $-CH_2CH_2SCH_3$, $-CH_2(CH_2)_2NH_2$, $-CH_2(CH_2)_3NH_2$, $-CH_2COOR'''$, $-CH_2CONH_2$, $-CH_2CH_2COOR'''$, $-CH_2CH_2CONH_2$, Phenyl, 4-Hydroxy- und 4-Methoxyphenyl, und Benzyl, wobei R"' die vorstehende Bedeutung besitzt. Besonders bevorzugte Reste R sind $-CH_3$, $-CH(CH_3)_2$, $-CH_2CH(CH_3)_2$, $-CH_2CH_2SCH_3$ und Benzyl.

Bevorzugte Reste R' sind lineare, verzweigte oder cyclische $C_{1-10}$-Alkyl- oder $C_{1-10}$-Alkoxyalkylreste, besonders bevorzugt sind $-CH_3$, $-CH_2OCH_3$, $-CH_2CH_3$, $-CH_2CH_2CH_3$ und $CH(CH_3)_2$.

Als optisch aktive Aminkomponente der allgemeinen Formel III* sind insbesondere optisch aktive 2-Aminocarbonsäureester in der S-Konfiguration zu nennen, da S-konfigurierte 2-Amino-carbonsäuren und deren Derivate in der Natur vorkommen. Beispiele hierfür sind (S)-Alanin-, (S)-Valin-, (S)-Leucin-, (S)-Methionin- und (S)-Phenylalanin-methylester.

Die optisch aktiven 2-Aminocarbonsäureester der allgemeinen Formel III* können nach literaturbekannten Methoden in Form ihrer Hydrochloride leicht aus den entsprechenden 2-Aminocarbonsäuren, Alkoholen und HCl-Gas hergestellt werden (Freudenberg et al., Ber. Dtsch. Chem. Ges. 63 (1930) 2380, Schwyzer et al., Helv. Chim. Acta 41 (1958) 1272, Grassmann et al., Chem. Ber. 91 (1958) 455, Rowlands und Young, J. Chem. Soc. 1952, 1937, Flowers und Reith, Biochem. J. 53 (1953) 657). Die Darstellung des Methioninmethylesterhydrochlorids kann mit Thionylchlorid erfolgen (Brenner et al., Helv. Chim. Acta 33 (1950) 568).

Die Umsetzung der racemischen Carbonsäuren der allgemeinen Formeln I oder II oder ihrer Derivate mit den optisch aktiven 2-Aminocarbonsäureestern kann direkt ohne weitere Zusätze oder in Gegenwart eines inerten Lösungsmittels wie Toluol, Tetrahydrofuran, Methyl-tert.-butylether, Ether oder chlorierter Kohlenwasserstoffe erfolgen. Bevorzugte Lösungsmittel sind Tetrahydrofuran und Methyl-tert.-butylether. Gegebenenfalls können bei der Umsetzung von Säurechloriden zusätzlich basische Hilfsstoffe wie Carbonate, beispielsweise Natriumcarbonat, Kaliumcarbonat oder Magnesiumcarbonat oder tertiäre Amine, beispielsweise Triethylamin oder Pyridin, eingesetzt werden. Beim Einsatz von tertiären Aminen wird vorzugsweise in Gegenwart von inerten Lösungsmitteln gearbeitet, um auch in Anwesenheit des entstehenden, nur wenig löslichen tert.-Aminhydrochlorids noch eine ausreichende Durchmischung zu gewährleisten.

Das molare Verhältnis von racemischer Carbonsäure bzw. deren Derivat zum optisch aktiven 2-Aminocarbonsäureester beträgt vorzugsweise zwischen 1:2 und 2:1, besonders bevorzugt werden die Reaktionspartner in äquimolaren Mengen eingesetzt. Die Umsetzung erfolgt unter Rühren vorzugsweise bei einer Temperatur zwischen 20 und 100 °C, insbesonders zwischen 20 und 70 °C. Die Reaktion kann nach Vorlage beider Reaktionspartner, Vorlage eines Reaktanden und Dosierung des zweiten Reaktanden oder Dosierung beider Reaktionspartner erfolgen. Vorzugsweise wird beim Arbeiten ohne Zusatz von tertiären Aminen eine Komponente vorgelegt und die zweite zudosiert. Bei Zusatz eines tertiären Amins wird dieses vorzugsweise zusammen mit dem optisch aktiven 2-Aminocarbonsäureester in einem Lösungsmittel vorgelegt und das Säurechlorid zudosiert. Die Reaktionsdauer beträgt in Abhängigkeit von der Reaktionstemperatur und der Rührintensität zwischen 2 und 20 Std.

Nach Abschluß der Reaktion kann beim Arbeiten ohne Zusatz von tertiären Aminen der Reaktionsansatz ohne weitere Aufarbeitung der Diastereomerentrennung zugeführt werden. Gegebenenfalls kann der Ansatz nach Reaktionsende mit einem inerten mit Wasser nicht mischbaren Lösungsmittel wie Methyl-tert.-butylether oder Toluol verdünnt werden und zur Entfernung von Säure mit Wasser oder Carbonatlösung behandelt werden. Beim Einsatz von tertiären Aminen ist es zweckmäßig, diese nach Reaktionsende in Form ihrer Hydrochloride zunächst abzufiltrieren und das Filtrat nach Waschen mit Wasser, verd. Salzsäure und Carbonatlösung der Diastereomerentrennung zuzuführen.

Die Diastereomerentrennung kann mittels Destillation, Chromatographie oder durch fraktionierte Kristallisation erfolgen. In einer bevorzugten Ausbildung des erfindungsgemäßen Verfahrens erfolgt die Diastereomerentrennung auf destillativem Weg.

Bei der gaschromatographischen Untersuchung der diastereomeren Carbonsäureamide der allgemeinen Formeln IV oder V,

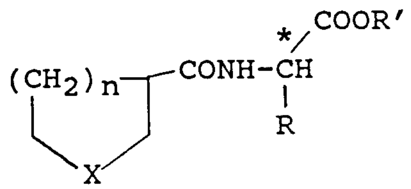

IV

oder

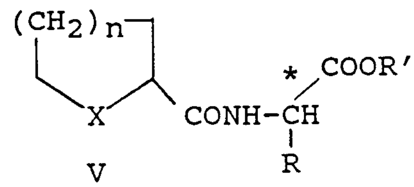

V

in denen n, X, R und R' vorstehende Bedeutung haben, wurden überraschenderweise erstaunlich große Unterschiede in den Retentionszeiten gefunden, die, wie bei den hier untersuchten Diastereomeren auch experimentell belegt, mit den Siedepunktsunterschieden direkt korrelieren. Somit eignet sich bei dem vorliegenden Verfahren eine vorausgehende gaschromatographische Untersuchung für die Planung der nachfolgenden präparativen destillativen Trennung (Druck/Temperatur, erforderliche Bodenzahl).

Da die Diasteromeren der Carbonsäureamide der allgemeinen Formeln IV oder V bei der gaschromatographischen Trennung stark unterschiedliche Retentionszeiten zeigen und daher auch große Siedepunktsunterschiede besitzen, kann die Trennung mit üblichen Destillationsapparaturen und Kolonnen durchgeführt werden. Bevorzugt sind Kolonnen, die sich für Vakuumdestillation eignen, wie beispielsweise Kolonnen mit regellosen Schüttungen (z.B. Füllkörpern) oder geordneten Einbauten (z.B. Gewebepackungen, Sulzer-Packungen), oder Drehbandkolonnen. Besonders bevorzugt sind Destillationsapparaturen mit einer Trennbodenzahl zwischen 1 und 100, insbesondere 5 und 60.

Die Destillation kann bei Atmosphärendruck oder unter vermindertem Druck ausgeführt werden. Bevorzugt sind Verfahren unter vermindertem Druck, da hierdurch das zu trennende Material aufgrund der Siedepunktsabsenkung weniger thermisch belastet wird. Bei Arbeiten unter vermindertem Druck sind Drücke zwischen 0.01 und 50 Torr bevorzugt, insbesondere Drücke zwischen 0.1 und 20 Torr. Die Kopftemperatur beträgt vorzugsweise 30 bis 180 °C, besonders bevorzugt 80 bis 160 °C. Die Sumpftemperatur beträgt vorzugsweise 50 bis 200 °C.

In einer weiteren Ausführungsform des erfindungsgemäßen Verfahrens kann die Trennung der Diastereomeren der allgemeinen Formeln IV oder V, wie bereits vorstehend beschrieben, mittels chromatographischer Methoden, insbesondere auf gaschromatographischem Weg, erfolgen. Hierfür geeignete Säulen sind beispielsweise Quarzkapillarsäulen mit Siliconen oder Polyethylenglycolen, z.B. SE 54 oder Carbowax 20M (Fa. Macherey-Nagel, W-5160 Düren-Rölsdorf) als stationäre Phasen bei Temperaturen zwischen 70 und 250 °C.

In einer weiteren Ausführungsform des erfindungsgemäßen Verfahrens können die Diastereomeren der allgemeinen Formeln IV oder V durch Kristallisation getrennt werden. Als Lösungsmittel können Kohlenwasserstoffe, wie beispielsweise Pentan, Hexan, Heptan, Cyclohexan oder Methylcyclohexan, Tetrachlorkohlenstoff oder auch Gemische der vorstehend genannten Lösungsmittel, oder Alkohole, beispielsweise Methanol oder Ethanol verwendet werden. Bevorzugte Lösungsmittel sind Pentan, Hexan, Methylcyclohexan und Methanol.

Vorzugsweise wird vor der Diastereomerentrennung durch Kristallisation die Estergruppe der Verbindungen der allgemeinen Formeln IV oder V in die freie Carbonsäuregruppe überführt, indem man mit Wasser

oder Wasser und Säure umsetzt. Bevorzugt ist Salzsäure bei molaren Säurekonzentrationen von 0.5 bis 3M. Besonders bevorzugt ist 1N Salzsäure bei Temperaturen zwischen 40 und 70 °C, insbesondere bei Temperaturen zwischen 50 und 60 °C. Es ist ebenfalls eine Umsetzung in alkalisch-wäßriger Lösung bei Temperaturen zwischen 30 und 60 °C möglich, z.B. unter Verwendung von 0.1 bis 2N NaOH oder Carbonatlösung. Alkalische Bedingungen sind aber sauren gegenüber weniger bevorzugt, weil dann für die Aufarbeitung zunächst angesäuert werden muß und die hierbei entstehenden Salze abgetrennt werden müssen. Als Zusätze, die die Löslichkeit der Verbindungen mit den allgemeinen Formeln IV und V bei der Umsetzung erhöhen, können wasserlösliche organische Lösungsmittel wie Tetrahydrofuran oder 1,4-Dioxan in Mengen bis zu 50 Vol-% oder Alkohole wie Methanol, Ethanol oder Isopropanol in Mengen bis zu 20 Vol-% verwendet werden. Die Reaktionszeiten liegen bei 12 Std. bis 4 Tagen. Unter den bevorzugten Bedingungen (1N HCl, 50-60 °C) betragen sie 2-3 Tage.

Nach der Umsetzung wird die saure Reaktionslösung zur Isolierung der freien Carbonsäure mit der allgemeinen Formel IV oder V (R' = H) eingedampft und zur Trennung der Diastereomeren aus einem geeigneten Lösungsmittel umkristallisiert. Geeignete Lösungsmittel umfassen Wasser, Wasser und Salzsäure, Methanol oder Ethanol oder deren Gemische mit Wasser, außerdem Kohlenwasserstoffe, Tetrachlorkohlenstoff oder Gemische aus den beiden zuletzt genannten Lösungsmitteln. Beispiele für geeignete Kohlenwasserstoffe sind Pentan, Hexan, Heptan, Cyclohexan, Methylcyclohexan, Xylol, Toluol, Benzol oder Mesitylen.

Zur Isolierung der jeweiligen optisch aktiven heterocyclischen Carbonsäuren der allgemeinen Formel I* oder II* wird nach erfolgter Diastereomerentrennung die Amidbindung gespalten. Die Spaltung erfolgt in wäßriger Phase unter Zugabe der 5- bis 50-fachen Gewichtsmenge Wasser. Durch Zugabe von Säure, bevorzugt Salzsäure, wird ein pH-Wert zwischen 1 und 6 eingestellt. Die Spaltung wird bei einer Temperatur von vorzugsweise 80 bis 200 °C, insbesonders 80 bis 120 °C, durchgeführt. Besonders bevorzugt ist die Spaltung in 1N Salzsäure bei einer Temperatur zwischen 100 und 120 °C, da unter diesen Bedingungen die optisch aktive 2-Aminocarbonsäure ohne Racemisierung zurückgewonnen werden kann.

Zur Beschleunigung der Spaltreaktion können gegebenenfalls noch Metallsalze in einer Menge von vorzugsweise 1 bis 20 Mol%, insbesondere 4 bis 6 Mol-%, jeweils bezogen auf die isolierte Fraktion der Carbonsäureamide, zugesetzt werden. Bevorzugt sind hierbei Chloride zweiwertiger Übergangsmetalle, beispielsweise $ZnCl_2$, $NiCl_2$ oder $CuCl_2$, insbesondere $ZnCl_2$. Die Dauer der Spaltreaktion beträgt im allgemeinen zwischen 2 und 10 Tagen.

Zur Aufarbeitung wird das Reaktionsgemisch nach der Spaltung bei einem pH-Wert von 1 bis 2 eingedampft und der Rückstand zur Abtrennung der optisch aktiven 2-Aminocarbonsäure in einem inerten Lösungsmittel wie Toluol, Tetrahydrofuran oder Methyl-tert.-butylether digeriert. Der unlösliche Rückstand besteht aus der optisch aktiven 2-Aminocarbonsäure in Form ihres Hydrochlorids, die auf diese Weise zurückgewonnen wird und, wie bereits beschrieben wieder in den entsprechenden Ester überführt werden kann. Die organische Phase wird nach dem Abdampfen des Lösungsmittels zur Reindarstellung der optisch aktiven Carbonsäure der allgemeinen Formel I* oder II* bei Drücken zwischen 0.01 und 20 Torr fraktioniert destilliert.

Die nach dem erfindungsgemäßen Verfahren hergestellten optisch aktiven Carbonsäuren eignen sich zur Verwendung bei der Herstellung flüssigkristalliner Verbindungen. Ferner kann die erfindungsgemäße Verfahrensweise dazu verwendet werden, mit Hilfe der erhaltenen optisch aktiven heterocyclischen Carbonsäuren optisch aktive Aminosäuren in hochreiner Form darzustellen.

Die Beispiele erläutern die Erfindung.

Beispiel 1

(Darstellung der heterocyclischen Carbonsäurechloride)

Zur Überführung in das Säurechlorid wurden 440 g (3.79 mol) Tetrahydrofuran-2-carbonsäure (Formel II, X = O, n = 1) in 400 ml Toluol innerhalb 2-3 Std. mit 580 g (4.88 mol) Thionylchlorid versetzt. Anschließend ließ man bei 65 °C noch 1 Std. nachreagieren und gewann das Reaktionsprodukt durch fraktionierte Destillation, Ausbeute: 400 g (82 %), Sdp. 70-72 °C/17-20 Torr (Lit. (Mooradian, J. Am. Chem. Soc. 71 (1949) 3372): 80-81 °C/30 Torr), IR (Film): 1800s (CO) $cm^{-1}$.

Analog wurden erhalten:

Tetrahydrothiophen-2-carbonsäurechlorid (Ausbeute: 91%, Sdp. 70 °C/1.5 Torr (Lit. (Wróbel und Hejchman, Synthesis 1987, 452): 113-114 °C/25 Torr), IR (Film): 1786s (CO) $cm^{-1}$)),

(2H)-Tetrahydropyran-2-carbonsäurechlorid (Ausbeute: 83%, Sdp. 52-56 °C/1.1 Torr, IR (Film): 1806 s (CO) $cm^{-1}$, $^1$H-NMR ($CDCl_3$): $\delta$ = 1.48-2.00 (m, 5H), 2.13 (m, 1H), 3.57 (mc, H-6), 4.07 und 4.27 (mc, H-6 und H-2)),
(2H)-Tetrahydrothiopyran-2-carbonsäurechlorid (Ausbeute: 56g (92%), Sdp. 75-80 °C/0.2 Torr, IR (Film): 2930m (CH), 2850w (CH), 1789s (CO) $cm^{-1}$, $^{1}$H-NMR ($CDCl_3$): $\delta$ = 1.56-1.98 (m, 4H), 2.00-2.19 (m, 1H), 2.19-2.31 (m, 1H), 2.41-2.61 (m, 1H), 2.72-2.91 (m, 1H), 3.79 (dd, CH)),
Tetrahydrofuran-3-carbonsäurechlorid (Ausbeute: 45%, Sdp. 55-58 °C/15 Torr, IR (Film): 1792s (CO) $cm^{-1}$, $^{1}$H-NMR ($CDCl_3$): $\delta$ = 2.1-2.5 (m, $CH_2$), 3.55 (mc, CH), 3.82 (mc, CH), 3.87-4.03 (m,$CH_2$), 4.08 (mc, CH)).

Beispiel 2

(Darstellung der heterocyclischen Carbonsäureamide der Formel IV oder V mit X = O,S und n = 1,2)

617 g (3.68 mol) (S)-Valinmethylesterhydrochlorid wurden in ca. 2.2 l Tetrahydrofuran suspendiert und mit 745 g (7.38 mol) Triethylamin versetzt. Unter Rühren wurden 451 g (3.35 mol) Tetrahydrofuran-2-carbonsäurechlorid in 200 ml THF zugetropft, wobei die Reaktionstemperatur bis auf 65 °C anstieg. Nach dem Ende der Zugabe wurde noch 12 Std. bei Raumtemperatur gerührt. Der Niederschlag wurde abgesaugt, gründlich mit Tetrahydrofuran gewaschen und die vereinigten organischen Phasen eingedampft. Der Rückstand wurde zur Entfernung von überschüssigem (S)-Valinmethylester in Ether gelöst, mit Wasser geschüttelt und wie üblich getrocknet. Die Vakuumdestillation liefert N-(2-Tetrahydrofuroyl)-(S)-valinmethylester (Formel V mit X = O, n = 1, R = $CH(CH_3)_2$ und R' = $CH_3$) als farblose Flüssigkeit, Sdp. 98-103 °C/0.01 Torr, Ausbeute: 611 g (80 %).

Nach dieser Vorschrift wurden auch die folgenden Carbonsäureamide erhalten:
N-(2-Tetrahydrofuroyl)-(S)-valinethylester (Formel V mit X = O, n = 1, R = $CH(CH_3)_2$ und R' = $C_2H_5$), Ausb. 87%, Sdp. 107-110 °C/0.01 Torr;
N-(2-Tetrahydrofuroyl)-(S)-alaninmethylester (Formel V mit X = O, n = 1, R = $CH_3$ und R' = $CH_3$), Sdp. 92-96 °C/0.01 Torr;
N-(2-Tetrahydrofuroyl)-(S)-alaninethylester (Formel V mit X = O, n = 1, R = $CH_3$ und R' = $C_2H_5$), Sdp. 103-107 °C;
N-(2-Tetrahydrofuroyl)-(S)-leucinmethylester (Formel V mit X = O, n = 1, R = $CH_2CH(CH_3)_2$ und R' = $CH_3$), Ausb. 80%, Sdp. 108-115 °C/0.01 Torr;
N-(2-Tetrahydrofuroyl)-(S)-methioninmethylester (Formel V mit X = O, n = 1, R = $CH_2CH_2SCH_3$ und R' = $CH_3$), Sdp. 153-156 °C/0.1 Torr;
N-(2-Tetrahydrofuroyl)-(S)-phenylalaninmethylester (Formel V mit X = O, n = 1, R = $CH_2$-$C_6H_5$ und R' = $CH_3$), Sdp. 162 °C/0.01 Torr;
N-(2-Tetrahydrothiophenoyl)-(S)-valinmethylester (Formel V mit X = S, n = 1, R = $CH(CH_3)_2$ und R' = $CH_3$), Schmp. 51-58 °C, Sdp. 135-144 °C/0.05 Torr;
N-(2-Tetrahydrothiophenoyl)-(S)-alaninethylester (Formel V mit X = S, n = 1, R = $CH_3$ und R' = $C_2H_5$), Schmp. 45-50 °C;
N-(2-Tetrahydrothiophenoyl)-(S)-leucinmethylester (Formel V mit X = S, n = 1, R = $CH_2CH(CH_3)_2$ und R' = $CH_3$), Ausb. 66%;
N-((2H)-2-Tetrahydropyranoyl)-(S)-valinmethylester (Formel V mit X = O, n = 2, R = $CH(CH_3)_2$ und R' = $CH_3$), Ausb. 80%, Sdp.120-125 °C;
N-((2H)-2-Tetrahydropyranoyl))-(S)-leucinmethylester (Formel V mit X = O, n = 2, R = $CH_2CH(CH_3)_2$ und R' = $CH_3$), Sdp. 116-120 °C/0.05 Torr;
N-((2H)-2-Tetrahydropyranoyl)-(S)-methioninmethylester (Formel V mit X = O, n = 2, R = $CH_2CH_2SCH_3$ und R' = $CH_3$), Ausb. 83%, Sdp. 152-156 °C/0.05 Torr;
N-((2H)-2-Tetrahydrothiopyranoyl)-(S)-valinmethylester (Formel V mit X = S, n = 2, R = $CH(CH_3)_2$ und R' = $CH_3$), Ausb. 73%, Schmp. 45-50 °C, Sdp. 123-132 °C/0.05 Torr;
N-((2H)-2-Tetrahydrothiopyranoyl)-(S)-valinethylester (Formel V mit X = S, n = 2, R = $CH(CH_3)_2$ und R' = $C_2H_5$), Ausb. 79%, Sdp. 147-155 °C/0.2 Torr;
N-((2H)-2-Tetrahydrothiopyranoyl)-(S)-leucinmethylester (Formel V mit X = S, n = 2, R = $CH_2CH(CH_3)_2$ und R' = $CH_3$);
N-(3-Tetrahydrofuroyl)-(S)-valinmethylester (Formel IV mit X = O, n = 1, R = $CH(CH_3)_2$ und R' = $CH_3$), Sdp. 107-109 °C/0.01 Torr;
N-(3-Tetrahydrofuroyl)-(S)-leucinmethylester (Formel IV mit X = O, n = 1, R = $CH_2CH(CH_3)_2$ und R' = $CH_3$).

Beispiel 3

(N-(2-Tetrahydrofuroyl)-(S)-valinmethylester)

30.4 g (0.226 mol) Tetrahydrofuran-2-carbonsäurechlorid wurden mit 41 g (0.246 mol) (S)-Valinmethylester-hydrochlorid in 50 ml Toluol unter Rühren auf 50 °C erwärmt bis zum Ende der HCl-Entwicklung (ca. 20 Std.). Es wurde mit wenig Wasser ausgeschüttelt und destilliert. Ausbeute an N-(2-Tetrahydrofuroyl)-(S)-valinmethylester (Formel V mit X = O, n = 1, R = $CH(CH_3)_2$ und R' = $CH_3$): 48.4 g (94 %).

Beispiel 4

(N-(2-Tetrahydrothiophenoyl)-(S)-valinmethylester)

Zu einer Suspension von 210 g (1.26 mol) (S)-Valinmethylester-hydrochlorid in 350 ml Toluol wurden bei 60 °C 134 g (0.89 mol) Tetrahydrothiophen-2-carbonsäurechlorid unter Rühren zugetropft. Nach beendeter Zugabe rührte man 20 Std. bei 70 °C weiter, dekantierte von festen Bestandteilen ab, schüttelte die Lösung zweimal mit je ca. 30 ml Wasser und destillierte. Ausb. 183 g (79%) N-(2-Tetrahydrothiophenoyl)-(S)-valinmethylester (Formel V mit X = S, n = 1, R = $CH(CH_3)_2$ und R' = $CH_3$), Schmp. 55-65 °C, Sdp. 115-128 °C/0.01 Torr.

Beispiel 5

Gaschromatographische Untersuchung der Carbonsäureamide der Formel IV und V

Die Carbonsäureamide der Formel IV und V wurden gaschromatographisch auf der unpolaren Siliconphase SE 54 (25 m Quarzkapillare der Fa. Macherey-Nagel, W 5160 Düren-Rölsdorf; Bedingungen: 2 min. isotherm bei 70 °C, Aufheizrate 12 °C/min bis 250 °C) und der polareren Phase Carbowax 20M (25 m Quarzkapillare der Fa. Macherey-Nagel, W 5160 Düren-Rölsdorf; Bedingungen: 70-230 °C mit einer Aufheizrate von 8 °C/min) untersucht. Die Unterschiede in den Retentionszeiten, siehe Tab. 1, dienten der Beurteilung des erforderlichen Destillationsaufwands.

Beispiel 6

(Destillative Trennung von N-(2-Tetrahydrofuroyl)-(S)-valinmethylester)

Die destillative Trennung des Carbonsäureamids aus Beispiel 3 der Formel V mit X = O, n = 1, R = $CH(CH_3)_2$ und R' = $CH_3$ wurde mit einer 1m-Füllkörperkolonne (Füllung: 4mm-Glasspiralen, Innendurchmesser 5 cm) mit einem Kolonnenkopf mit automatischem Flüssigkeitsteiler vorgenommen. Rücklaufverhältnis 5:1 bis ca. 10:1; pro Std. werden ca. 50 ml Destillat abgenommen. Es wurde bei Drücken von 0.05 bis 0.01 Torr gearbeitet.

Aus 2.7 kg Carbonsäureamid wurden bei der Destillation unter den angegebenen Bedingungen die folgenden Fraktionen erhalten:

a) niedriger-siedendes RS-Diastereomeres (V*(X = O, n = 1, R = $CH(CH_3)_2$ und R' = $CH_3$)); opt. Reinheit in Klammern: 86 g (99.9%), 673 g (99.0%, $[\alpha]_D^{23}$ = + 16.27 in Subst.), 220 g (98.5%), 192 g (97.0%);

b) Mittelfraktion: 422 g Mischung;

c) höher-siedendes SS-Diastereomeres (V*(X = O, n = 1, R = $CH(CH_3)_2$ und R' = $CH_3$)): 235 g (99.0%, $[\alpha]_D^{23}$ = -10.3 in Subst.), 419 g (99.8%).

Siedepunkte siehe Tabelle 1, analytische Daten siehe Tabelle 2, spektroskopische Daten siehe Tabelle 3

Beispiel 7

(Destillative Trennung von N-(2-Tetrahydrofuroyl)-(S)-valinmethylester)

An einer 1m-Füllkörperkolonne (Füllung 4mm-Wilsonspiralen; Innendurchmesser 3 cm, Rücklaufverhältnis 10:1, Druck 0.01 Torr) lieferten 620 g Carbonsäureamid aus Beispiel 3 der Formel V mit X = O, n = 1, R = $CH(CH_3)_2$ und R' = $CH_3$ bei der Destillation 206 g des niedriger-siedenden RS-Diastereomeren der Formel V* mit X = O, n = 1, R = $CH(CH_3)_2$ und R' = $CH_3$ (opt. Reinheit 95%) und 147 g des höher-siedenden SS-Diastereomeren der Formel V* mit X = O, n = 1, R = $CH(CH_3)_2$ und R' = $CH_3$ (opt. Reinheit 98%).

Beispiel 8

(Destillative Trennung von N-(2-Tetrahydrofuroyl)-(S)-valinmethylester)

An einer 1m-Füllkörperkolonne (Füllung: Sulzer-Packungen aus Edelstahl, Innendurchmesser 5 cm, Rücklaufverhältnis 10:1, Druck 0.01 Torr) lieferten 1100 g Carbonsäureamid aus Beispiel 3 der Formel V mit X = O, n = 1, R = $CH(CH_3)_2$ und R' = $CH_3$ bei der Destillation 468 g des niedriger-siedenden RS-Diastereomeren der Formel V* mit X = O, n = 1, R = $CH(CH_3)_2$ und R' = $CH_3$(opt. Reinheit 85%) und 137 g des höhersiedenden SS-Diastereomeren der Formel V* mit X = O, n = 1, R = $CH(CH_3)_2$ und R' = $CH_3$ (opt. Reinheit 97%).

Beispiel 9

(Destillative Trennung von N-(2-Tetrahydrothiophenoyl)-(S)-valinmethylester)

183 g Carbonsäureamid aus Beispiel 4 der Formel V mit X = S, n = 1, R = $CH(CH_3)_2$ und R' = $CH_3$ wurden an einer beheizten 1m-Füllkörperkolonne (Durchmesser 5 cm, 5 mm-Glaswendeln, Druck 0.05 Torr) destilliert. Man erhielt die niedriger siedende optisch aktive Komponente der Formel V* mit X = S, n = 1, R = $CH(CH_3)_2$ und R' = $CH_3$ (R,S-Diastereomeres) in einer optischen Reinheit von 91% (gaschromatographisch).

Beispiel 10

(Destillative Trennung von N-(2-Tetrahydrothiophenoyl)-(S)-valinmethylester)

Das Carbonsäureamid (Beispiel 2) der Formel V mit X = S, n = 1, R = $CH(CH_3)_2$ und R' = $CH_3$ wurde an einer Drehbandkolonne destilliert. Bei 0.05 Torr und einer Siedetemperatur von 110-117 °C erhielt man das niedrigersiedende (RS)-Diastereomere in einer optischen Reinheit von 100% (gaschromatographisch). $[\alpha]_D^{23}$ = -14.07, c = 1.0 ($CHCl_3$). Schmp. 60-62 °C.

Beispiel 11

(Destillative Trennung von N-((2H)-2-Tetrahydrothiopyranoyl)-(S)-valinmethylester)

105 g Carbonsäureamid (Beispiel 2) der Formel V mit X = S, n = 2, R = $CH(CH_3)_2$ und R' = $CH_3$ wurden an einer beheizten Widmer-Kolonne (Länge 30 cm) bei 0.01 Torr destilliert. Man erhielt die niedriger siedende (Sdp. 134 °C) optisch aktive Komponente der Formel V* mit X = S, n = 2, R = $CH(CH_3)_2$ und R' = $CH_3$ in einer optischen Reinheit von 62%.

Beispiel 12

(Destillative Trennung)

Durch Destillation an einer 1m-Drehbandkolonne (Teflonband, Rücklaufverhältnis ca. 30:1, Druck ca. 0.01 Torr) wurden die optisch aktiven Carbonsäureamide V* mit X = O, n = 1, R = $CH(CH_3)_2$ und R' = $C_2H_5$; V* mit X = O, n = 1, R = $CH_3$ und R' = $CH_3$; V* mit X = O, n = 1, R = $CH_3$ und R' = $C_2H_5$; V* mit X = O, n = 1, R = $CH_2CH(CH_3)_2$ und R' = $CH_3$; V* mit X = O, n = 2, R = $CH(CH_3)_2$ und R' = $CH_3$ (niedriger siedendes Diastereomeres: $[\alpha]_D^{22}$ = + 51.4, c = 1.02 ($CHCl_3$); höhersiedendes Diastereomeres: $[\alpha]_D^{21}$ = -16.7, c = 1.02 ($CHCl_3$)) und IV* mit X = O, n = 1, R = $CH(CH_3)_2$ und R' = $CH_3$ jeweils aus den entsprechenden Diastereomerengemischen IV und V (hergestellt nach Beispiel 2) erhalten.

Die erzielten optischen Reinheiten und Siedepunkte befinden sich in Tabelle 1. Die analytischen bzw. spektroskopischen Daten der getrennten Substanzpaare IV* und V* befinden sich in Tabelle 2 bzw. Tabelle 3.

Beispiel 13

(Isolierung von optisch aktiver (R)-Tetrahydrofuran-2-carbonsäure)

100 g (0.437 mol) Carbonsäureamid (hergestellt nach Beispiel 6) der Formel V* mit X = O, n = 1, R = CH-$(CH_3)_2$ und R' = $CH_3$ mit einem Diastereomerenverhältnis SS:RS = 1.3:98.7 wurden in 400 ml 1N HCl bei 100 °C unter Zusatz von ca. 3 g (22 mmol) $ZnCl_2$ ca. 4 Tage gerührt. Die Reaktionslösung wurde eingedampft und der Rückstand mit Methyl-tert.-butylether (MTBE) digeriert. Die filtrierte MTBE-Phase wurde fraktioniert destilliert. Man erhielt 36 g (78%) (R)-Tetrahydrofuran-2-carbonsäure (Formel II* mit X = O, n = 1) mit Sdp. 83 °C/0.3 Torr, Lit.(Pasto und Serve, J. Am. Chem. Soc. 87 (1965) 1515): 97-100 °C/1.05 Torr.$[\alpha]_D^{27}$ = +33.3, c = 1.23 ($CHCl_3$); Lit. (Bélanger et al., Can. J. Chem. 61 (1983) 1383): 30.1, c = 1.21 ($CHCl_3$) für das (S)-Enantiomere.
$[\alpha]_D^{23}$ = + 33.5, $[\alpha]_D^{27}$ = + 35.1, c = 1.07 ($CHCl_3$); Lit. (Bélanger et al.): + 30.4, c = 1.01 ($CHCl_3$).
Zur Bestimmung des Enantiomerenverhältnisses wurde die erhaltene Tetrahydrofuran-2-carbonsäure mit $LiAlH_4$ zum 2-Tetrahydrofurfurylalkohol reduziert und dieser gaschromatographisch (chirale Stationärphase Chiral-XE- 60-S-Val der Fa. Chrompak, München) untersucht. Es ergab sich R:S = 98.7:1.3.
Der Filterrückstand der MTBE-Phase, der aus (S)-Valin-hydrochlorid besteht, wurde im Vakuum getrocknet und wog dann 43 g (70%). Die optische Reinheit des zurückgewonnenen Valins wurde nach Umsetzung mit Methanol/HCl und mit Trifluoressigsäureanhydrid gaschromatographisch auf Chiral-XE-60-S-Val bestimmt. Es ergab sich S:R = 98.3:1.7.

Beispiel 14

(Isolierung von optisch aktiver Tetrahydrothiophen-2-carbonsäure)

5.2 g Carbonsäureamid (isoliert gemäß Beispiel 9) der Formel V* mit X = S, n = 1, R = $CH(CH_3)_2$ und R' = $CH_3$ (niedriger-siedendes RS-Diastereomeres mit einer opt. Reinheit von 91%) wurden in 100 ml 1N Salzsäure unter Zusatz von 0.3 g Zinkchlorid 30 Std. auf 100 °C erhitzt. Anschließend wurde eingedampft, mit Toluol digeriert, filtriert und destilliert. Man erhielt 1.75 g (70%) (R)-Tetrahydrothiophen-2-carbonsäure (Formel II* mit X = S und n = 1) mit Sdp. 93-94 °C/0.1 Torr. $[\alpha]_D^{22}$ = - 112.4 (c = 0.442 (96 proz. Ethanol)) (Lit. (Claeson und Jonsson): $[\alpha]_D^{25}$ = -154.9 (c = 0.47 (96 proz. Ethanol)).

Beispiel 15

(Isolierung von optisch aktiver (2H)-Tetrahydropyran-2-carbonsäure)

5.4 g Carbonsäureamid (isoliert gemäß Beispiel 12) der Formel V* mit X = O, n = 2, R = $CH(CH_3)_2$ und R' = $CH_3$ (niedrigersiedendes RS-Diastereomeres mit einer opt. Reinheit von 54%) wurden in 100 ml 1N Salzsäure unter Zusatz von 0.35 g Zinkchlorid 40 Std. auf 100 °C erhitzt. Anschließend wurde eingedampft, mit Toluol digeriert, filtriert und destilliert. Man erhielt 2.25 g (85%) (R)-(2H)-Tetrahydropyran-2-carbonsäure (Formel II* mit X = O und n = 2) mit Sdp. 79-80 °C/0.1 Torr. $[\alpha]_D^{23}$ = + 15.98 (c = 1.02 ($CHCl_3$))

Beispiel 16

(Isolierung von optisch aktiver (2H)-Tetrahydrothiopyran-2-carbonsäure)

2.0 g Carbonsäureamid (isoliert gemäß Beispiel 11) der Formel V* mit X = S, n = 2, R = $CH(CH_3)_2$ und R' = $CH_3$ wurden in 20 ml 2N Salzsäure unter Zusatz von 0.2 g Zinkchlorid 40 Std. auf 100 °C erhitzt. Man erhielt (2H)-Tetrahydrothiopyran-2-carbonsäure (Formel II* mit X = S und n = 2).
IR (KBr): 2928s (CH); 1704s (CO) $cm^{-1}$ - $^1$H-NMR ($CDCl_3$): $\delta$ = 1.42-1.61 (m, 1H), 1.71-2.21 (m, 5H), 2.51-2.69 (m, 1H), 2.79-2.90 (m, 1H), 3.56 (dd, CH), 10.9 (s, breit, COOH)

Beispiel 17

(Kristallisation von N-(2-(S)-Tetrahydrofuroyl)-(S)-valin)

23.0 g (0.10 mol) Carbonsäureamid (hergestellt gemäß Beispiel 2) der Formel V mit X = O, n = 1, R = $CH(CH_3)_2$ und R' = $CH_3$ wurden in 100 ml 1N HCl bei 50-60 °C ca. 3 Tage gerührt und die Reaktionslösung dann auf ca. 50 ml eingedampft und abkühlen gelassen. Hierbei kristallisierte das SS-Diastereomere V* mit X = O, n = 1, R = $CH(CH_3)_2$ und R' = H in einer Ausbeute von 12.7 g (59 %) aus. Die Diastereomerenreinheit wurde nach Umsetzung zum Methylester gaschromatographisch bestimmt und betrug >99.9%. Schmp. 143-149 °C, IR (KBr): 3400m(NH), 2960m (CH), 1733s (CO), 1625s (CO) $cm^{-1}$, $^1$H-NMR ($CDCl_3$): $\delta$ = 0.99 (2d, $2CH_3$), 1.92, 2.07, 2.30 (3mc,je 2H), 3.94(mc,2H), 4.43, 4.54 (2mc, je 1H), 7.23 (2s, breit, NH), 9.6 (s,breit, COOH)

Tabelle 1: GC-Retentionszeiten für die Carbonsäureamide der Formeln IV* und V* und die bei der präparativen Trennung gemessenen Siedepunkte und erzielten optischen Reinheiten.

| Verbindungspaare | $t_{ret1}$ a) | $t_{ret2}$ b) | Sdp. c) | %* d) | Type e) |
|---|---|---|---|---|---|
| V*(X=O, n=1, R= | 1. 10.54 | 16.68 | 98 | 99(RS) | FKK(6) |
| $CH(CH_3)_2$, R'=$CH_3$) | 2. 10.90 | 17.58 | 103 | 99.8(SS) | FKK(6) |
| V*(X=O, n=1, R= | 1. 11.21 | 17.16 | 107 | 99(RS) | DBK(12) |
| $CH(CH_3)_2$, R'=$C_2H_5$) | 2. 11.55 | 17.95 | 110 | 99.5(SS) | DBK(12) |
| V*(X=O, n=1, R= | 1. 9.29 | 16.33 | 92 | 99(RS) | DBK(12) |
| $CH_3$, R'=$CH_3$) | 2. 9.53 | 16.93 | 95 | 99.5(SS) | DBK(12) |
| V*(X=O, n=1, R= | 1. 10.09 | 16.68 | 103 | 99(RS) | DBK(12) |
| $CH_3$, R'=$C_2H_5$) | 2. 10.32 | 17.28 | 107 | 99.5(SS) | DBK(12) |
| V*(X=O, n=1, R= | 1. 11.56 | 18.57 | 108 | 99.8(RS) | DBK(12) |
| $CH_2CH(CH_3)_2$,R'=CH3) | 2. 11.86 | 19.07 | 115 | 95.1(SS) | DBK(12) |
| V*(X=O, n=1, R= | 1. 14.23 | 21.13 | | | |
| $(CH_2)_2SCH_3$,R'=$CH_3$) | 2. 14.35 | 21.98 | | | |
| V*(X=O, n=1, R= | 1. 15.06 | 25.51 | | | |
| $CH_2$-$C_6H_5$, R'=$CH_3$) | 2. 15.29 | 27.05 | | | |
| V*(X=S, n=1, R= | 1. 12.52 | 21.06 | 104 f) | 91.0 | FKK(9) |
| $CH(CH_3)_2$, R'=$CH_3$) | 2. 12.88 | 22.19 | | | |
| | | | 110 -117f) | 100.0 | DBK(10) |
| V*(X=S,n=1, R= | 1. 11.92 | 20.98 | | | |
| $CH_3$, R'=$C_2H_5$) | 2. 12.17 | 21.74 | | | |

Tabelle 1: (Fortsetzung)

| Verbindungspaare | | $t_{ret1}$ a) | $t_{ret2}$ b) | Sdp. c) | %* d) | Type e) |
|---|---|---|---|---|---|---|
| V*(X=S, n=1, R= | 1. | 13.14 | 22.39 | | | |
| $CH_2CH(CH_3)_2$, R'=$CH_3$) | 2. | 13.33 | 23.33 | | | |
| V*(X=O, n=2, R= | 1. | 11.68 | 18.62 | 77 | 90 | DBK(12) |
| $CH(CH_3)_2$, R'=$CH_3$) | 2. | 11.88 | 19.08 | 82 | 94 | DBK(12) |
| V*(X=O, n=2, R= | 1. | 12.43 | 19.82 | | | |
| $CH_2CH(CH_3)_2$, R'=$CH_3$) | 2. | 12.54 | 20.05 | | | |
| V*(X=O, n=2, R= | 1. | 15.00 | 25.48 | | | |
| $(CH_2)_2SCH_3$, R'=$CH_3$) | 2. | 15.03 | 25.83 | | | |
| V*(X=S, n=2, R= | 1. | 13.00 | 21.82 | 134 | 62 | WK(11) |
| $CH(CH_3)_2$, R'=$CH_3$) | 2. | 13.40 | 23.13 | f) | | |
| V*(X=S, n=2, R= | 1. | 13.65 | 21.89 | | | |
| $CH(CH_3)_2$, R'=$C_2H_5$) | 2. | 14.01 | 23.14 | | | |
| V*(X=S, n=2, R= | 1. | - | 21.04 | | | |
| $CH_3$, R'=$CH_3$) | 2. | - | 22.42 | | | |
| V*(X=S, n=2, R= | 1. | 13.65 | 21.78 | | | |
| $CH_3$, R'=$C_2H_5$) | 2. | 14.01 | 22.69 | | | |
| V*(X=S), n=2, R= | 1. | 13.73 | 23.42 | | | |
| $CH_2CH(CH_3)_2$, R'=$CH_3$) | 2. | 13.93 | 24.49 | | | |
| IV*(X=O, n=1, R= | 1. | 11.50 | 20.51 | 107 | 73 | DBK(12) |
| $CH(CH_3)_2$, R'=$CH_3$) | 2. | 11.57 | 20.67 | 109 | 72 | DBK(12) |

Tabelle 1: (Fortsetzung)

| Verbindungspaare | | $t_{ret1}$ a) | $t_{ret2}$ b) | Sdp. c) | %* d) | Typ e) |
|---|---|---|---|---|---|---|
| IV*(X=O, n=1, R= | 1. | 12.34 | 22.37 | | | |
| $CH_2CH(CH_3)_2$,R'=$CH_3$) | 2. | 12.35 | 22.54 | | | |

a) GC-Retentionszeit auf SE 54, 25 m Quarzkapillare, 2 min isotherm bei 70°C, Aufheizrate 12°C/min bis 250°C;

b) GC-Retentionszeit auf Carbowax 20M, 25m Quarzkapillare, 70-230°C mit Aufheizrate von 8°C/min;

c) bei einem Druck von ca. 0.01 bis 0.05 Torr;

d) optische Reinheit in % bei präparativer Trennung;

e) FKK: Füllkörperkolonne, DBK: 1m-Drehbandkolonne, WK: Widmer-Kolonne, Zahl in Klammern gibt Nr. des Beispiels an;

f) das 2. Diastereomere ist im Sumpf angereichert.

Tabelle 2: Analytische Daten optisch aktiver Carbonsäureamide der Formeln IV* und V*.

| Produkt | Konfig.[a)] | | Elementaranalyse |
|---|---|---|---|
| V*(X=O, n=1, R= $CH(CH_3)_2$, R'=$CH_3$) | RS (≥99) SS (≥99) | $C_{11}H_{19}NO_4$ (229.3) | Ber. C 57.62 H 8.35 N 6.11<br>Gef. C 57.73 H 8.32 N 6.16<br>Gef. C 57.39 H 8.11 N 6.28 |
| V*(X=O, n=1, R= $CH(CH_3)_2$, R'=$C_2H_5$) | RS[a)] (99.9) SS (99.9) | $C_{12}H_{21}NO_4$ (243.3) | Ber. C 59.24 H 8.70 N 5.76<br>Gef. C 59.41 H 8.70 N 5.88<br>Gef. C 59.26 H 8.75 N 5.77 |
| V*(X=O, n=1, R= $CH_2CH(CH_3)_2$, R'=$CH_3$) | RS (99.8) SS (95) | $C_{12}H_{21}NO_4$ (243.3) | Ber. C 59.24 H 8.70 N 5.76<br>Gef. C 59.02 H 8.57 N 5.94<br>Gef. C 59.30 H 8.56 N 5.93 |
| V*(X=O, n=1, R= $CH_3$, R'=$C_2H_5$) | RS (99.8) SS (99.0) | $C_{10}H_{17}NO_4$ (215.3) | Ber. C 55.80 H 7.96 N 6.51<br>Gef. C 55.73 H 8.01 N 6.87<br>Gef. C 56.03 H 7.95 N 6.94 |
| V*(X=S, n=1, R= $CH(CH_3)_2$, R'=$CH_3$) | RS (100) | | |
| V*(X=O, n=2, R= $CH(CH_3)_2$, R'=$CH_3$) | RS (90.0) SS (94.0) | $C_{12}H_{21}NO_4$ (243.3) | Ber. C 59.24 H 8.70 N 5.76<br>Gef. C 59.46 H 8.97 N 5.73<br>Gef. C 59.22 H 8.96 N 6.10 |
| IV*(X=O, n=1, R= $CH(CH_3)_2$, R'=$CH_3$ | 1.[b)] (73) 2. (72) | $C_{11}H_{19}NO_4$ (229.3) | Ber. C 57.62 H 8.35 N 6.11<br>Gef. C 56.04 H 7.96 N 5.96<br>Gef. C 57.32 H 8.27 N 6.15 |

a) Konfiguration, gaschromatographisch bestimmte optische Reinheit in Klammern,

b) "1." bezieht sich auf das Diastereomere mit niedrigerem Siedepunkt.

Tabelle 3: Spektroskopische Daten der Carbonsäureamide der Formeln IV* und V*

| Verbindungspaar | IR(Film)/$\gamma$($cm^{-1}$)[a] | $^1$H-NMR($CDCl_3$)/$\delta$(ppm) |
|---|---|---|
| V*(X=O, n=1, R= $CH(CH_3)_2$, R'=$CH_3$) | 1. 3405w, 2960m, 2873m, 1743s, 1687s | 0.93 (t,J=7Hz,$CH_3$), 1.93 (mc,2H), 2.0-2.4 (m, 3H), 3.75 (s,$OCH_3$), 3.90, 4.00 (2mc,2H-5), 4.41(dd,$\alpha$-CH), 4.53 (dd,H-2), 7.10, 7.15 (2s,breit,NH) |
| | 2. 3405w, 2960m, 2873m, 1743s, 1687s | 0.90, 1.91, 2.0-2.4, 3.75, 3.96 (mc,2H-5), 4.39, 4.54, 7.13, 7.19[b] |
| V*(X=O, n=1, R= $CH(CH_3)_2$, R'=$C_2H_5$) | 1. 3415w, 2963w, 1740s, 1688s | 0.91 (t,J=7Hz,$CH_3$), 1.26 (t,J=7.5Hz,$CH_3$), 1.91 (mc,2H), 2.0-2.4 (m,3H), 3.90, 4.05 (2mc,2H-5), 4.20 (mc,$CH_2$), 4.40 (dd,$\alpha$-CH), 4.50 (dd,H-2), 7.10, 7.15 (2s,breit,NH) |
| | 2. 3412w, 2963m, 1740s, 1686s | 0.90, 1.26, 1.8-2.4 (m,5H) 3.95 (mc,2H-5), 4.20(q,J= 7.5Hz,$CH_2$), 4.38, 4.50, 7.15, 7.20[b] |
| V*(X=O, n=1, R= $CH_3$, R'=$CH_3$) | 1. 3402w, 3320w, 2980w, 2950m, 2878w, 1745s, 1672s | 1.41 (d,J=7.5Hz,$CH_3$), 1.93 (mc,2H), 2.0-2.4 (m, 3H), 3.75 (s,$OCH_3$), 3.83-4.08 (mc,2H-5), 4.36(dd, $\alpha$-CH), 4.60 (mc,H-2), 7.18 (breit,NH) |
| | 2. s.o. | 1.43, 1.94, 2.0-2.4, 3.75, 3.96, 4.39, 4.63[b] |

Tabelle 3: (Fortsetzung)

| Verbindungspaar | IR(Film)/γ ($cm^{-1}$)[a] | $^1$H-NMR($CDCl_3$)/δ (ppm) |
| --- | --- | --- |
| V*(X=O, n=1, R= $CH_3$, R'=$C_2H_5$) | 1. 3404w, 2980m, 1740s, 1674s | 1.28 (t,J=7Hz,CH3), 1.43 (d,J=7.5Hz,$CH_3$), 1.91(m, 2H), 2.0-2.4 (m,3H), 3.90, 4.00 (2mc,2H-5), 4.19 (q, J=7Hz,CH2), 4.36 (dd,α-CH) 4.39 (mc,H-2), 7.10, 7.15 (2s,breit,NH) |
| | 2. 3404w, 2981m, 1740s, 1675s | 1.28, 1.41, 1.7-2.4 (m,5H) 3.93 (mc,2H-5), 4.21, 4.35 4,54, 7.20, 7.30[b] |
| V*(X=O, n=1, R= $CH_2CH(CH_3)_2$,R'=$CH_3$) | 1. 3400w, 2950m, 1743s, 1675s | 0.95 (mc,2$CH_3$), 1.50-1.73 (m,3H), 1.93 (mc,$CH_2$), 2.05-2.15 (m,CH),2.17-2.32 (m,CH), 3.73 (s,$OCH_3$), 3.90, 4.03, 4.37, 4.60 (4mc,4H),6.97 (d,breit,NH) |
| | 2. 3400w, 2950m, 1745s, 1670s | 0.93 (mc,2$CH_3$), 1.53-1.78 (m,2CH), 1.79-2.13 (m, 3CH), 2.20-2.36 (m,CH), 3.72 (s, $OCH_3$), 3.83-4.00 (2CH), 4.35 (mc,CH),4.53-4.67(m,CH),7.10 (d,breit, NH) |
| V*(X=O, n=1, R= $(CH_2)_2SCH_3$, R'=$CH_3$) | 1. 2945m, 1741s, 1672s | 1.78-2.35 (m,6H), 2.10 (s, $SCH_3$), 2.50 (mc,$CH_2$),3.73 (s,$OCH_3$), 3.90, 3.97,4.37, 4.69 (4mc,4H), 7.17 (m,breit,NH) |
| | 2. wie 1. | wie 1. |

Tabelle 3: (Fortsetzung)

| Verbindungspaar | IR(Film)/$\gamma$($cm^{-1}$)[a] | $^1$H-NMR($CDCl_3$)/$\delta$(ppm) |
|---|---|---|
| V*(X=O, n=1, R= $CH_2$-$C_6H_5$, R'=$CH_3$) | 1. 3400w, 2973m, 1742s, 1675s | 1.52-2.32 (m,4H), 2.97-3.23 (m,$CH_2$), 3.70 (s,$OCH_3$), 3.72-3.93 (m,$CH_2$), 4.30 (mc,1H), 4.85 (m,1H), 6.97-7.33 (m, 5H und NH) |
| | 2. wie 1. | 3.73 (s,$OCH_3$), weitere Signale wie 1. |
| V*(X=S, n=1, R= $CH(CH_3)_2$, R'=$CH_3$) | 1. | 0.89-1.02 (m,2$CH_3$), 1.81-2.41 (m,2$CH_2$), 2.96, 3.06 (2mc,2CH), 3.73(s,$OCH_3$), 3.97 (mc,CH), 4.51(mc,CH), 7.40 (d,breit,NH) |
| | 2. | 3.74(s,$OCH_3$), 7.45 (d, breit,NH), weitere Signale wie 1. |
| V*(X=S, n=1, R= $CH_3$, R'=$CH_3$) | 1. | 1.28 (t,J=8Hz,$CH_3$), 1.83-2.42 (m,4H), 2.90, 3.03 (2mc,$CH_2$), 3.93 (mc,CH), 4.20 (q, J=8Hz,$CH_2$), 4.52 (mc,CH), 7.45 (d,breit,NH) |
| | 2. | 1.29 (t,J=8Hz,$CH_3$), 4.21 (q,J=8Hz,$CH_2$) |
| V*(X=S, n=1, R= $CH_2CH(CH_3)_2$,R'=$CH_3$) | 1. | 0.92 (mc,2$CH_3$), 1.50-1.77 (m,3H), 1.82-2.24 (m,3H), 2.33 (mc,CH), 2.82, 3.10 (2mc, $CH_2$), 3.73 (s,$OCH_3$), 3.93, 4.57 (2mc,2CH), 7.26 (d, breit,NH) |
| | 2. | 3.72(s,$OCH_3$),7.38(d,breit, NH), weit.Signale wie 1. |

Tabelle 3: (Fortsetzung)

| Verbindungspaar | IR(Film)/γ($cm^{-1}$)a) | $^1H$-NMR($CDCl_3$)/δ(ppm) |
|---|---|---|
| V*(X=O, n=2, R= $CH(CH_3)_2$, R'=$CH_3$) | 1. 3418w, 2958m, 1742s, 1686s | 0.93 (mc, $2CH_3$), 1.33-1.67 (m, 2 $CH_2$), 1.89 (mc, CH), 2.06 (mc, CH), 2.17 (sept, CH), 3.50 (mc, CH), 3.74 (s, $OCH_3$), 3.83 (mc, CH), 4.09 (mc, CH), 4.53 (mc, CH) 7.00(d, breit, J=10Hz, NH) |
| | 2. wie 1. | 0.95 (mc, $2CH_3$), 1.28-1.67 (m, 2 $CH_2$), 1.90 (mc, CH), 2.12 (mc, CH), 2.17 (sept, CH), 3.49 (mc, CH), 3.74 (s, $OCH_3$), 3.80 (mc, CH), 4.07(mc, CH), 4.53 (mc, CH) 7.03(d, breit, J=10Hz, NH) |
| V*(X=O, n=2, R= $CH_2CH(CH_3)_2$, R'=$CH_3$) | 1. 3410m, 2950s, 1746s, 1680s | 0.94 (d,J=8Hz,$2CH_3$), 1.3-1.8 (m,6H), 1.90(mc, CH), 2.09 (mc,CH), 3.47, 3.80, 4.07, 4.63 (4mc,4CH), 3.73 (s,$OCH_3$), 6.88 (m,breit,NH) |
| | 2. wie 1. | wie 1. |
| V*(X=O, n=2, R= $(CH_2)_2SCH_3$, R'=$CH_3$) | 1. 3390m, 2942m, 1743s, 1660s | 1.28-1.67(m,6H), 1.83-2.26 (m,2H), 2.10 (s,$SCH_3$),2.52 (mc,$CH_2$), 3.48, 3.80, 4.07, 4.72 (4mc,4H), 3.74(s,$OCH_3$) 7.15 (d,breit,NH) |
| | 2. wie 1. | wie 1. |

Tabelle 3: (Fortsetzung)

| Verbindungspaar | IR(Film)/γ($cm^{-1}$)a) | $^1H$-NMR($CDCl_3$)/δ(ppm) |
|---|---|---|
| V*(X=S, n=2, R= $CH(CH_3)_2$, R'=$CH_3$) | 1. 3280m, 2921m 1745s | 0.90 (mc,$2CH_3$), 1.42-2.98 (m,10H), 3.40 (mc,CH), 3.78 (s,$OCH_3$), 4.54 (mc, CH), 7.38 (d,breit,NH) |
| | 2. | 3.80 (s,$OCH_3$), 7.50(d, breit,NH) |
| V*(X=S, n=2, R= $CH_2CH(CH_3)_2$,R'=$CH_3$) | 1. 2950m, 2923m 1742s, 1652s | 0.99 (mc,$2CH_3$), 1.51-2.98 (m,11H), 3.40 (mc,CH), 3.76 (s,$OCH_3$), 4.65 (mc,CH), 7.25 (d,breit,NH) |
| | 2. | 3.80 (s,$OCH_3$), 7.50 (d, breit,NH) |
| IV*(X=O, n=1, R= $CH(CH_3)_2$, R'=$CH_3$) | 1. 3275m, 2959m, 1742s, 1654s | 0.91(m,$2CH_3$),2.18(m,3CH), 3.00(mc,CH),3.72(s,$OCH_3$), 3.75-4.00 (m,3CH), 4.57 (mc,CH), 6.13 (d,breit,NH) |
| | 2. wie 1. | wie 1. |
| IV*(X=O, n=1, R= $CH_2CH(CH_3)_2$, R'=$CH_3$) | 3300s, 2952m 1748s, 1795s | 0.97 (m,$2CH_3$), 1.74-1.73 (m,3H), 2.17 (mc,$CH_2$),2.97 (mc,CH), 3.75(s,$OCH_3$),3.78 -4.00 (m,$2CH_2$), 4.62 (mc, CH), 6.38 (d,breit,NH)c) |

a) NH-, CH- und CO-Banden;
b) Zuordnung siehe 1. Diastereomeres;
c) Diastereomerengemisch

## Patentansprüche

1. Verfahren zur Herstellung optisch aktiver Carbonsäuren der allgemeinen Formeln I* oder II*,

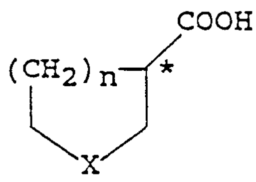

I*

oder

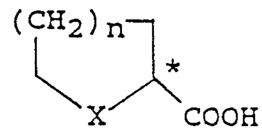

II*

in denen X ein Sauerstoff- oder Schwefelatom und n 1 oder 2 ist, dadurch gekennzeichnet, daß eine racemische Carbonsäure I oder II oder deren Derivate mit einem optisch aktiven 2-Aminocarbonsäureester zu den diastereomeren Carbonsäureamiden umgesetzt wird, die Diastereomeren getrennt werden und nach Spaltung der Amidbindung die optisch aktiven Carbonsäuren der allgemeinen Formeln I* oder II* isoliert werden.

2. Verfahren nach Anspruch 1, dadurch gekennzeichnet, daß als racemische Carbonsäuren I oder II solche der allgemeinen Formeln

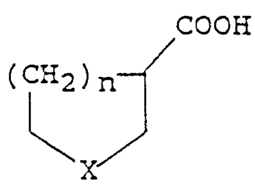

I

oder

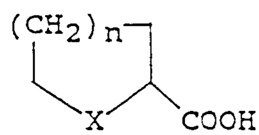

II

mit X = O oder S und n = 1 oder 2 eingesetzt werden.

3. Verfahren nach Anspruch 1, dadurch gekennzeichnet, daß als Derivate der racemischen Carbonsäuren I oder II deren Säurehalogenide, Ester, Anhydride, oder Amide eingesetzt werden.

4. Verfahren nach Anspruch 1, dadurch gekennzeichnet, daß als Derivate der racemischen Carbonsäuren I oder II deren Säurechloride eingesetzt werden.

5. Verfahren nach Anspruch 1, 2, 3 oder 4, dadurch gekennzeichnet, daß als optisch aktive 2-Aminocarbonsäureester (S)- oder (R)-2-Aminocarbonsäureester der allgemeinen Formel III*,

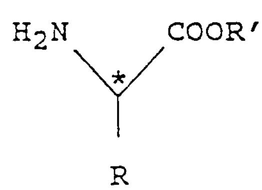

III*

eingesetzt werden, wobei der Rest R ein linearer, verzweigter oder cyclischer $C_{1-6}$-Alkylrest ist, bei dem gegebenenfalls ein Kohlenstoffatom durch ein Sauerstoff- oder Schwefelatom oder durch den Rest >NR''ersetzt ist, wobei R'' einen Methyl- oder Ethylrest bedeutet oder R ein linearer, verzweigter oder cyclischer $C_{1-6}$-Alkylrest ist, der mit den Resten -OH, -SH, $-SCH_3$, $-NH_2$, -COOR''', $-CONH_2$ oder durch eine Phenylgruppe, die gegebenenfalls mit $-CH_3$, -OH oder $-OCH_3$ substituiert ist, substituiert ist,

wobei R''' eine Methyl-, Ethyl-, n-Propyl- oder i-Propylgruppe bedeutet, oder der Rest R eine Phenylgruppe bedeutet, die unsubstituiert oder mit $-CH_3$, -OH oder - $OCH_3$ substituiert ist; und der Rest R' ein linearer, verzweigter oder cyclischer $C_{1-20}$-Alkyl- oder $C_{1-20}$-Alkoxyalkylrest, der gegebenenfalls mit einer Phenylgruppe substituiert ist oder ein Phenylrest ist.

**6.** Verfahren nach Anspruch 5, dadurch gekennzeichnet, daß der Rest R ein linearer oder verzweigter $C_{1-6}$-Alkylrest, $-CH_2OH$, $-CHOH(CH_3)$, $-CH_2SH$, $-CH_2CH_2SCH_3$, $-CH_2(CH_2)_2NH_2$, $-CH_2(CH_2)_3NH_2$, $-CH_2COOR'''$, $-CH_2CONH_2$, $-CH_2CH_2COOR'''$, - $CH_2CH_2CONH_2$, Phenyl, 4-Hydroxy- und 4-Methoxyphenyl, oder Benzyl-Rest ist.

**7.** Verfahren nach Anspruch 6, dadurch gekennzeichnet, daß der Rest R $-CH_3$, $-CH(CH_3)_2$, $-CH_2CH(CH_3)_2$, $-CH_2CH_2SCH_3$ oder ein Benzylrest ist.

**8.** Verfahren nach Anspruch 5 bis 7, dadurch gekennzeichnet, daß der Rest R' ein linearer, verzweigter oder cyclischer $C_{1-10}$-Alkyl- oder $C_{1-10}$-Alkoxyalkylrest ist.

**9.** Verfahren nach Anspruch 5 bis 8, dadurch gekennzeichnet, daß der Rest R' die Bedeutung $-CH_3$, $-CH_2OCH_3$, $-CH_2CH_3$, $-CH_2CH_2CH_3$ oder $CH(CH_3)_2$ hat.

**10.** Verfahren nach Anspruch 1 bis 9, dadurch gekennzeichnet, daß als optisch aktive Aminkomponente der allgemeinen Formel III* optisch aktive 2-Aminocarbonsäureester in der S-Konfiguration eingesetzt werden.

**11.** Verfahren nach Anspruch 1 bis 10, dadurch gekennzeichnet, daß die Trennung der diastereomeren Carbonsäureamide der Formeln IV und V

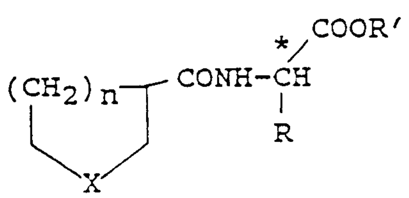

oder

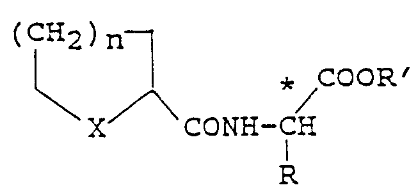

IV

V

mittels Destillation, Chromatographie oder fraktionierter Kristallisation durchgeführt wird.

**12.** Verfahren nach Anspruch 11, dadurch gekennzeichnet, daß die Diastereomerentrennung mittels Destillation erfolgt.

**13.** Verfahren nach Anspruch 12, dadurch gekennzeichnet, daß die Destillation unter vermindertem Druck durchgeführt wird.

**14.** Verfahren nach Anspruch 11, dadurch gekennzeichnet, daß die Trennung der Diastereomeren mittels Gaschromatographie erfolgt.

**15.** Verfahren nach Anspruch 11, dadurch gekennzeichnet, daß die Diastereomeren durch Kristallisation getrennt werden.

**16.** Verfahren nach Anspruch 15, dadurch gekennzeichnet, daß die Estergruppen der diastereomeren Carbonsäureamide vor deren Trennung durch fraktionierte Kristallisation in die freien Carbonsäuregruppen umgewandelt werden.

**17.** Verfahren nach Anspruch 1 bis 16, dadurch gekennzeichnet, daß zur Isolierung der optisch aktiven Carbonsäuren der Formel I* oder II* nach der Diastereomerentrennung die Amidbindung in wäßriger Phase bei einer Temperatur von 80 bis 200 °C gespalten wird.

**18.** Verfahren nach Anspruch 17, dadurch gekennzeichnet, daß die Spaltung in 1N Salzsäure bei einer Temperatur zwischen 100 und 120 °C durchgeführt wird.

**19.** Verfahren nach Anspruch 17 oder 18, dadurch gekennzeichnet, daß zur Beschleunigung der Spaltreaktion noch Metallsalze in einer Menge von 1 bis 20 Mol%, bezogen aus die isolierte Fraktion der Carbonsäureamide, zugesetzt werden.

**20.** Verfahren nach Anspruch 1 bis 19, dadurch gekennzeichnet, daß zur Reindarstellung der optisch aktiven Carbonsäuren der Formel I* oder II* das Reaktionsgemisch nach der Spaltung eingedampft wird, der Rückstand zur Rückgewinnung der optisch aktiven 2-Aminocarbonsäure in einem inerten Lösungsmittel digeriert wird und die organische Phase nach dem Abdampfen des Lösungsmittels fraktioniert destilliert wird.

## Claims

**1.** Process for the preparation of optically active carboxylic acids of the general formulae [sic] I* or II*

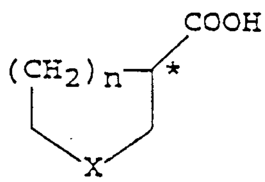

or

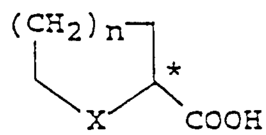

I* II*

in which X is an oxygen or sulphur atom and n is 1 or 2, characterized in that a racemic carboxylic acid I or II or its derivatives is reacted with an optically active 2-aminocarboxylic acid ester to give the diastereomeric carboxylic acid amides, the diastereomers are separated and, after cleavage of the amide bond, the optically active carboxylic acids of the general formula I* or II* are isolated.

**2.** Process according to Claim 1, characterized in that the racemic carboxylic acid I or II employed is that of the general formula

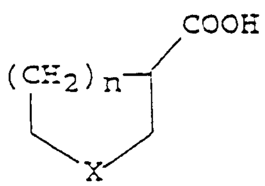

or

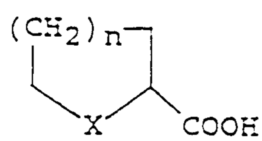

I II

with X = O or S and n = 1 or 2.

**3.** Process according to Claim 1, characterized in that the derivatives of the racemic carboxylic acids [sic] I or II employed are its acid halides, esters, anhydrides or amides.

**4.** Process according to Claim 1, characterized in that the derivatives of the racemic carboxylic acids [sic] I or II employed are its acid chlorides.

**5.** Process according to Claim 1, 2, 3 or 4, characterized in that the optically active 2-amino-carboxylic acid esters employed are the (S)- or (R)-2-aminocarboxylic acid esters of the general formula III*

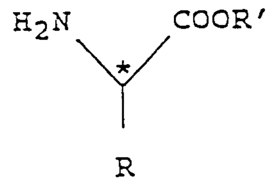

III*

in which the radical R is a linear, branched or cyclic $C_{1-6}$-alkyl radical in which a carbon atom is optionally replaced by an oxygen or sulphur atom or by the >NR" radical, where R" denotes a methyl or ethyl radical or R is a linear, branched or cyclic $C_{1-6}$-alkyl radical which is substituted by the radicals -OH, -SH, $-SCH_3$, $-NH_2$, -COOR"', $-CONH_2$ or by a phenyl group which is optionally substituted by $-CH_3$, -OH or $-OCH_3$, where R' denotes a methyl, ethyl, n-propyl or i-propyl group, or the radical R denotes a phenyl group which is unsubstituted or substituted by $-CH_3$, -OH or $-OCH_3$; and the radical R' is a linear, branched or cyclic $C_{1-20}$-alkyl- or $C_{1-20}$-alkoxyalkyl radical which is optionally substituted by a phenyl group, or is a phenyl radical.

**6.** Process according to Claim 5, characterized in that the radical R is a linear or branched $C_{1-6}$-alkyl radical, $-CH_2OH$, $-CHOH(CH_3)$, $-CH_2SH$, $-CH_2CH_2SCH_3$, $-CH_2(CH_2)_2NH_2$, $-CH_2(CH_2)_3NH_2$, $-CH_2COOR'''$, $-CH_2CONH_2$, $-CH_2CH_2COOR'''$, $-CH_2CH_2CONH_2$, phenyl, 4-hydroxy- and 4-methoxyphenyl or benzyl radical.

**7.** Process according to Claim 6, characterized in that the radical R is $-CH_3$, $-CH(CH_3)_2$, $-CH_2CH(CH_3)_2$, $-CH_2CH_2SCH_3$ or a benzyl radical.

**8.** Process according to Claims 5 to 7, characterized in that the radical R' is a linear, branched or cyclic $C_{1-10}$-alkyl or $C_{1-10}$-alkoxyalkyl radical.

**9.** Process according to Claims 5 to 8, characterized in that the radical R' has the meaning $-CH_3$, $-CH_2OCH_3$, $-CH_2CH_3$, $-CH_2CH_2CH_3$ or $CH(CH_3)_2$.

**10.** Process according to Claims 1 to 9, characterized in that the optically active amine component of the general formula III* employed is optically active 2-aminocarboxylic acid ester in the S configuration .

**11.** Process according to Claims 1 to 10, characterized in that the diastereomeric carboxylic acid amides of the formulae IV and V

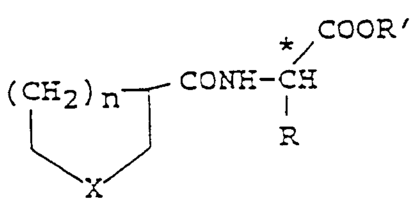

or

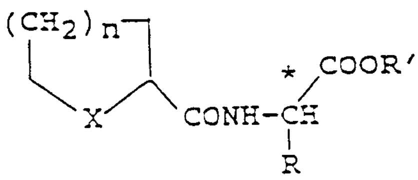

IV

V

are separated by means of distillation, chromatography or fractional crystallization.

**12.** Process according to Claim 11, characterized in that the diastereomer separation is carried out by means of distillation.

**13.** Process according to Claim 12, characterized in that the distillation is carried out under reduced pressure.

14. Process according to Claim 11, characterized in that the diastereomers are separated by means of gas chromatography.

15. Process according to Claim 11, characterized in that the diastereomers are separated by crystallization.

16. Process according to Claim 15, characterized in that the ester groups of the diastereomeric carboxylic acid amides are converted into the free carboxylic acid groups before separation thereof by fractional crystallization.

17. Process according to Claims 1 to 16, characterized in that, to isolate the optically active carboxylic acids of the formula I* or II*, the amide bond is cleaved in the aqueous phase at a temperature of 80 to 200 °C after the diastereomer separation.

18. Process according to Claim 17, characterized in that the cleavage is carried out in 1N hydrochloric acid at a temperature between 100 and 120 °C.

19. Process according to Claim 17 or 18, characterized in that metal salts are also added in an amount of 1 to 20 mol%, relative to the isolated fraction of the carboxylic acid amides, to accelerate the cleavage reaction.

20. Process according to Claims 1 to 20, characterized in that, to prepare the pure optically active carboxylic acids of the formula I* or II*, the reaction mixture is evaporated after cleavage, the residue is digested in an inert solvent to recover the optically active 2-aminocarboxylic acid and the organic phase is fractionally distilled after evaporation of the solvent.

## Revendications

1. Un procédé de préparation d'acides carboxyliques optiquement actifs de formule générale I* ou II* ci-dessous

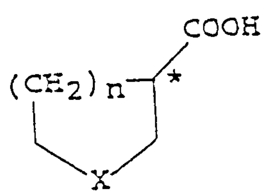

I*

ou

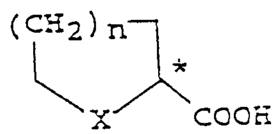

II*

X désignant un atome d'oxygène ou de soufre et n le nombre 1 ou 2, procédé caractérisé en ce que l'on fait réagir un acide carboxylique racémique I ou II ou un dérivé de celui-ci avec un ester d'acide 2-aminocarboxylique optiquement actif pour former les amides carboxyliques diastéréomères, puis on sépare les diastéréomères et après avoir dissocié la liaison amide on isole les acides carboxyliques optiquement actifs de formule I* ou II*.

2. Procédé selon la revendication 1, caractérisé en ce que les acides carboxyliques racémiques I ou II sont des acides de formule

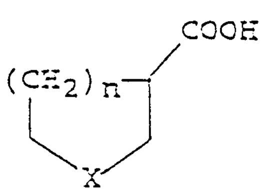

I

ou

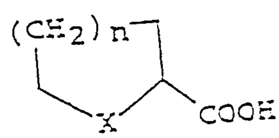

II

avec X = O ou S et n = 1 ou 2.

3. Procédé selon la revendication 1, caractérisé en ce que l'on utilise comme dérivés des acides carboxyliques racémiques I ou II leurs halogénures, esters, anhydrides ou amides.

4. Procédé selon la revendication 1, caractérisé en ce que les dérivés des acides racémiques sont les chlorures.

5. Procédé selon la revendication 1, 2, 3 ou 4, caractérisé en ce que l'on utilise comme esters d'acides 2-aminocarboxyliques optiquement actifs des esters d'acides 2-aminocarboxyliques (S) ou (R) de formule générale III* ci-dessous

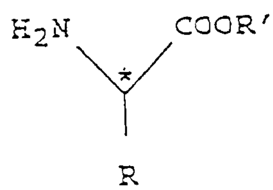

III*

dans laquelle R désigne un alkyle en $C_1$-$C_6$ linéaire, ramifié ou cyclique, dont éventuellement un atome de carbone peut être remplacé par un atome d'oxygène ou de soufre ou par un radical >NR'', R'' étant le radical méthyle ou éthyle, ou bien R désigne un alkyle en $C_1$-$C_6$, linéaire, ramifié ou cyclique, avec comme substituants des radicaus -OH, -SH, $-SCH_3$, $-NH_2$, -COOR''', $-CONH_2$ ou un groupe phényle, éventuellement substitué par un groupe $-CH_3$, -OH ou $-OCH_3$, R''' étant un groupe méthyle, éthyle, n-propyle ou i-propyle, ou encore R désigne un groupe phényle sans substituants ou avec un groupe $-CH_3$, -OH ou $-OCH_3$ comme substituants, et R' désigne un alkyle en $C_1$-$C_{20}$ ou un alcoxyalkyle en $C_1$-$C_{20}$, linéaire, ramifié ou cyclique, avec éventuellement un groupe phényle comme subtituant, ou bien le radical phényle.

6. Procédé selon la revendication 5, caractérisé en ce que le radical R est un radical alkyle en $C_{1-6}$ linéaire ou ramifié, $-CH_2OH$, $-CHOH(CH_3)$, $-CH_2SH$, $-CH_2CH_2SCH_3$, $-CH_2(CH_2)_2NH_2$, $-CH_2(CH_2)_3NH_2$, $-CH_2COOR'''$, $-CH_2CONH_2$, $CH_2CH_2COOR'''$, $CH_2CH_2CONH_2$, phényle, 4-hydroxy ou 4-méthoxyphényle, ou benzyle.

7. Procédé selon la revendication 6, caractérisé en ce que le radical R est un radical $-CH_3$, $-CH(CH_3)_2$, $-CH_2CH(CH_3)_2$, $-CH_2CH_2SCH_3$ ou benzyle .

8. Procédé selon les revendications 5 à 7, caractérisé en ce que le radical R' est un radical alkyle en $C_{1-10}$ ou alcoxyalkyle en $C_{1-10}$, linéaire, ramifié ou cyclique.

9. Procédé selon les revendications 5 à 8, caractérisé en ce que le radical R' est un radical $-CH_3$, $-CH_2OCH_3$, $-CH_2CH_3$, $-CH_2CH_2CH_3$ ou $-CH(CH_3)_2$.

10. Procédé selon les revendications 1 à 9, caractérisé en ce que le composant aminé optiquement actif de formule III* est un ester d'acide 2-aminocarboxylique optiquement actif ayant la configuration S.

11. Procédé selon les revendications 1 à 10, caractérisé en ce que l'on sépare les amides carboxyliques diastéréomères de formules IV et V

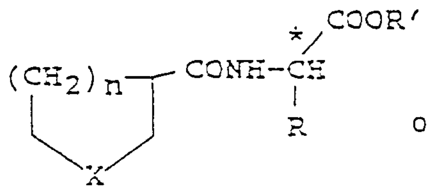

IV

ou

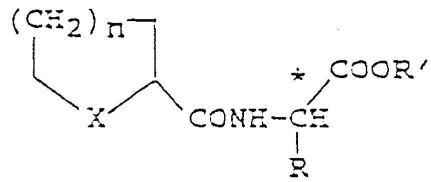

V

par distillation, chromatographie ou cristallisation fractionnée.

**12.** Procédé selon la revendication 11, caractérisé en ce que l'on sépare les diastéréomères par distillation.

**13.** Procédé selon la ravendication 12, caractérisé en ce que l'on effectue la distillation sous pression réduite.

**14.** Procédé selon la revendication 11, caractérisé en ce que l'on sépare les diastéréomères par chromatographie en phase gazeuse.

**15.** Procédé selon la revendication 11, caractérisé en ce que l'on sépare les diastéréomères par cristallisation.

**16.** Procédé selon la revendication 15, caractérisé en ce que, avant leur séparation par cristallisation fractionnée, on transforme les groupes esters des amides carboxyliques diastéréomères en groupes carboxyliques libres.

**17.** Procédé selon les revendications 1 à 16, caractérisé en ce que pour isoler les acides carboxyliques optiquement actifs de formule I* ou II* après la séparation des diastéréomères, on dissocie la liaison amide en phase aqueuse à une température de 80 à 200 ° C.

**18.** Procédé selon la revendication 17, caractérisé en ce que l'on effectue la dissociation dans de l'acide chlorhydrique normal à une température de 100 à 120 ° C.

**19.** Procédé selon la revendication 17 ou 18, caractérisé en ce que pour accélérer la réaction de dissociation on ajoute des sels de métaux dans une proportion de 1 à 20 Mol% par rapport à la fraction isolée de l'amide carboxylique.

**20.** Procédé selon les revendications 1 à 19, caractérisé en ce que, pour obtenir à l'état pur les acides carboxyliques optiquement actifs de formule I* ou II*, après la dissociation on concentre le mélange de réaction par évaporation, on fait digérer la matière restante dans un solvant inerte pour en récupérer l'acide 2-aminocarboxylique optiquement actif, puis après évaporation du solvant on soumet la phase organique à une distillation fractionnée.